(19) Europäisches Patentamt
European Patent Office
Office européen des brevets

(11) EP 2 233 161 A2

(12) EUROPEAN PATENT APPLICATION

(43) Date of publication:
**29.09.2010 Bulletin 2010/39**

(51) Int Cl.:
***A61L 27/18*** *(2006.01)* ***A61L 27/52*** *(2006.01)*

(21) Application number: **10250605.2**

(22) Date of filing: **26.03.2010**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA ME RS**

(30) Priority: **27.03.2009 US 412751**

(71) Applicant: **Confluent Surgical Inc.**
**Waltham, Massachusetts 02451 (US)**

(72) Inventor: **Bennett, Steven L.**
**Cheshire, CT 06410 (US)**

(74) Representative: **Soames, Candida Jane**
**Edwards Angell Palmer**
**& Dodge Innovations LLP**
**10 Carlton Crescent**
**Southampton SO15 2EZ (GB)**

(54) **Low-swelling biocompatible hydrogels**

(57) Some aspects of the present disclosure relate to methods for treating a tissue by forming a low-swelling biodegradable hydrogel in situ adherent to the tissue. In embodiments the hydrogel exhibits negative swelling, i.e., shrinking. Such treatments may be utilized to in cosmetic or reconstructive surgery, in sphincter augmentation, treating nerve inflammation, and the like.

EP 2 233 161 A2

## Description

CROSS-REFERENCE TO RELATED APPLICATIONS

This application is a continuation-in-part of U.S. Patent Application Serial No. 11/714,028 filed on March 5, 2007, the entire disclosure of which is incorporated by reference herein.

TECHNICAL FIELD OF THE INVENTION

The present disclosure is related to surgical treatments using hydrogels, in embodiments bioabsorbable, covalently-crosslinked hydrogels.

BACKGROUND

Hydrogels may be used in the body for applications such as sealing, adhesion prevention, or drug delivery. Hydrogels can exhibit a generally high degree of swelling when hydrated.

Certain medical applications, however, do not tolerate a high degree of swelling. For instance, GELFOAM® absorbable gelatin (Pharmacia & Upjohn, Kalamazoo, MI) is a water-insoluble, porous, pliable form of gelatin for application to bleeding surfaces as a hemostatic. However, GELFOAM® is not suited for applications around the vertebral column and is contra-indicated for laminectomy procedures and for use near foramina in bone, once hemostasis is achieved. This contraindication exists because GELFOAM® may swell after absorbing physiological fluids and produce nerve damage by pressure within confined bony spaces. The packing of GELFOAM®, particularly within bony cavities, should be avoided, since swelling may interfere with normal function and/or possibly result in compression necrosis of surrounding tissues.

Excessive swelling may also be undesirable in cosmetic and reconstructive surgical applications, including wrinkle filling, as well as sphincter augmentation and the introduction of hydrogels into areas of a defined volume, including the carpal tunnel.

Hydrogels having low swelling thus remain desirable for many applications, including cosmetic surgery and the filling of voids in tissue.

SUMMARY

Some aspects of the present disclosure relate to methods for treating tissue by forming a low-swelling biodegradable hydrogel in situ. In embodiments the hydrogel exhibits negative swelling, i.e., shrinking.

For example, in embodiments, a method of the present disclosure may include contacting tissue with a first synthetic precursor possessing first functional groups; and contacting the first precursor with a second synthetic precursor including a multi-armed precursor possessing a core possessing from about 3 to about 12 arms, the arms each including a polyethylene glycol having a molecular weight from about 250 to about 5000 and possessing second functional groups at the ends thereof, wherein the first functional groups crosslink with the second functional groups thereby forming a hydrogel which swells from about -50% to about 50%.

The hydrogel may rapidly crosslink to form a gel, in embodiments, in less than about 10 seconds after contacting the first precursor with the second precursor.

In some embodiments, the hydrogel of the present disclosure may shrink by a weight decrease of from about 1% to about 50%.

In embodiments, bioactive agents and/or visualization agents may be administered with the first precursor and/or second precursor.

In embodiments, the methods of the present disclosure may be utilized to treat contour deficiencies of the skin. Such contour deficiencies may include deficiencies found on skin located on a portion of the body such as cheeks, nose, ears, and skin adjacent an eye. In embodiments, such contour deficiencies in the skin may include frown lines, worry lines, wrinkles, crow's feet, marionette lines, stretch marks, internal scars, external scars, and combinations thereof.

In other embodiments, the methods of the present disclosure may be utilized to treat nerves, tissue adjacent the nerves, combinations thereof, and the like. In some cases, the methods of the present disclosure may include introducing a hydrogel composition in a space between a nerve and its adjacent tissue, including bony tissue. Thus, methods of the present disclosure may include, in embodiments, introducing the hydrogel into the interior of the carpal tunnel.

The invention may be described by reference to the following numbered paragraphs:-

1. The use of a first synthetic precursor possessing first functional groups in the preparation of a medicament for

use in a method of treating tissue which comprises the steps of:

contacting tissue with the first precursor and with a second synthetic precursor comprising a multi-armed precursor possessing a core possessing from about 3 to about 12 arms, the arms each comprising a polyethylene glycol having a molecular weight from about 250 to about 5000 and possessing second functional groups at the ends thereof,

wherein the first functional groups crosslink with the second functional groups thereby forming a hydrogel which swells from about -50% to about 50%.

2. The synthetic precursor of paragraph 1, wherein the hydrogel is crosslinked to form a gel in less than about 10 seconds after contacting the first precursor with the second precursor and/or the method of claim 1, wherein the first functional groups comprise nucleophiles and the second functional groups comprise electrophiles and/or the method of claim 1, wherein the first synthetic precursor is selected from the group consisting of dilysines, trilysines, and tetralysines.

3. The synthetic precursor of paragraph 1 or paragraph 2, wherein the first synthetic precursor comprises an oligopeptide sequence of no more than about five residues comprising at least two lysine groups.

4. The synthetic precursor of any preceding paragraph, wherein the core of the second precursor is selected from the group consisting of polyethers, polyamino acids, proteins, and polyols; preferably wherein the core of the second precursor is selected from the group consisting of polyethylene glycol, polyethylene oxide, polyethylene oxide-co-polypropylene oxide, co-polyethylene oxide copolymers, polyvinyl alcohol, polyvinyl pyrrolidinone, poly(amino acids), dextran, proteins, derivatives thereof, and combinations thereof.

5. The synthetic precursor of of paragraph 1, wherein the multi-armed precursor possesses from about 4 to about 8 arms.

6. The synthetic precursor of paragraph 1, wherein the combined weight of the arms of the multi-armed precursor is from about 750 to about 20000.

7. The synthetic precursor of paragraph 1, wherein the combined weight of the arms of the multi-armed precursor is from about 5000 to about 18000.

8. The synthetic precursor of paragraph 1, further comprising administering a bioactive agent with the first precursor and second precursor.

9. The synthetic precursor of paragraph 1, further comprising administering a visualization agent with the first precursor and second precursor; preferably wherein the visualization agent comprises a dye selected from the group consisting of FD&C Blue #1, FD&C Blue #2, FD&C Blue #3, D&C Green #6, methylene blue, and combinations thereof.

10. The synthetic precursor of paragraph 1, wherein the hydrogel shrinks by a weight decrease of from about 1% to about 50% or the method of claim 1, wherein the hydrogel shrinks by a weight decrease of from about 5% to about 30%.

11. The synthetic precursor of paragraph 1, wherein the tissue comprises contour deficiencies of skin.

12. The synthetic precursor of paragraph 11, wherein the contour deficiencies in the skin are found on skin located on a portion of the body selected from the group consisting of cheeks, nose, ears, and skin adjacent an eye; preferably wherein the contour deficiencies in the skin are selected from the group consisting of frown lines, worry lines, wrinkles, crow's feet, marionette lines, stretch marks, internal scars, external scars, and combinations thereof.

13. The synthetic precursor of paragraph 1, wherein the tissue comprises a nerve, tissue adjacent a nerve, or a space between a nerve and adjacent tissue.

14. The synthetic precursor of paragraph 13, wherein the space adjacent the nerve comprises the interior of the carpal tunnel.

## BRIEF DESCRIPTION OF THE DRAWINGS

Various embodiments of the present disclosure will be described herein below with reference to the figures wherein:

Figure 1 is a graph depicting the degree of swelling of hydrogels of the present disclosure formed with multi-armed precursors having differing numbers of arms and differing combined molecular weights of the arms; and

Figure 2 is a graph depicting the compressive strength of hydrogels of the present disclosure formed with multi-armed precursors having differing numbers of arms and differing combined molecular weights of the arms.

## DETAILED DESCRIPTION

Hydrogels are described herein that may be suitable for use in areas where little or low swelling is desired.

These hydrogels have good adhesion to tissues, can be formed in-situ, are optionally biodegradable, and exhibit low-swelling after placement so that soft tissues will not be unduly compressed when the hydrogel is placed in an area of defined volume, for example the vertebral column or carpal tunnel. Nerves, in particular, may be vulnerable when a conventional hydrogel swells and compresses a nerve against a bone. Thus, low-swelling hydrogel compositions described herein may create new therapeutic possibilities for trea0074ing tissues around or adjacent nerves, including in bony areas and/or adjacent bony tissue.

Hydrogels can be useful aids in surgical procedures for use, for example, as hemostats, sealants, protective barriers, and the like. The creation of hydrogels in situ in a patient may enable the creation of a hydrogel that coats tissue, conforms to its shape, and fills/conforms to a three dimensional space. Such materials should possess mechanical properties adequate to withstand strains caused by movement of the patient, shifting of tissues, hydrostatic forces present in the tissue, and the like. At the same time, a high water content can be useful for biocompatibility.

*Hydrogel Systems Overview*

Certain hydrogel properties can be useful, such as adhesion to a variety of tissues, fast setting times to enable a surgeon to accurately and conveniently place the hydrogels, high water content for biocompatibility, mechanical strength for use in sealants, and/or toughness to resist destruction after placement. Synthetic materials that are readily sterilized and avoid the dangers of disease transmission involved in the use of natural materials may thus be used. Indeed, certain in situ polymerizable hydrogels made using synthetic precursors are within the purview of those skilled in the art, e.g., as used in commercially available products such as FOCALSEAL® (Genzyme, Inc.), COSEAL® (Angiotech Pharmaceuticals), and DURASEAL® (Confluent Surgical, Inc). Other known hydrogels include, for example, those disclosed in U.S. Patent Nos. 6,656,200; 5,874,500; 5,543,441; 5,514,379; 5,410,016; 5,162,430; 5,324,775; 5,752,974; and 5,550,187.

The swelling of COSEAL® and DURASEAL® has been measured using an in vitro model in comparison to fibrin sealant (Campbell et al., Evaluation of Absorbable Surgical Sealants: In vitro Testing, 2005). Over a three day test, COSEAL® swelled an average of about 558% by weight, DURASEAL® increased an average of about 98% by weight, and fibrin sealant swelled by about 3%. Assuming uniform expansion along all axes, the percent increase in a single axis was calculated to be 87%, 26%, and 1% for COSEAL®, DURASEAL®, and fibrin sealant, respectively. Hydrogels with less swelling may be desirable for applications at or near the vertebral column, in areas having a defined volume, and in applications where excessive swelling should be avoided, e.g., sphincter augmentation, wrinkle filling, anastomotic sealing, sealing in and around the eye, and the like. Fibrin sealant is a proteinaceous glue that has adhesive, sealing, and mechanical properties that are inferior to COSEAL®, DURASEAL®, and other hydrogels disclosed herein. Further, it is typically derived from biological sources that are potentially contaminated, is cleared from the body by mechanisms distinct from this class of hydrogels, and typically requires refrigeration while stored.

In situ polymerizable hydrogels of the present disclosure may be made from precursors. The precursor may be a monomer or a macromer. One type of suitable precursor may have a functional group that is ethylenically unsaturated. An ethylenically unsaturated functional group may be polymerized using an initiator to start the reaction. Precursors with at least two ethylenically unsaturated functional groups may form crosslinked polymers. Some compositions have certain precursors with only one such functional group and additional crosslinker precursors with a plurality of functional groups for crosslinking the precursors. Ethylenically unsaturated functional groups may be polymerized by various techniques, including free radical, condensation and/or addition polymerization. Hydrogels may be formed from one precursor (as by free radical polymerization), two precursors, or made with three or more precursors, with one or more of the precursors participating in crosslinking to form the hydrogel.

Another type of precursor that may be utilized has a functional group that may be an electrophile or nucleophile. Electrophiles react with nucleophiles to form covalent bonds. Covalent crosslinks or bonds refer to chemical groups formed by reaction of functional groups on different polymers that serve to covalently bind the different polymers to each other. In certain embodiments, a first set of electrophilic functional groups on a first precursor may react with a second set of nucleophilic functional groups on a second precursor. When the precursors are mixed in an environment that permits reaction (for example, as relating to pH or solvent), the functional groups react with each other to form covalent bonds and join the precursors together. The precursors become crosslinked when at least some of the precursors can react with more than one other precursor. For instance, a precursor with two functional groups of a first type may be reacted with a crosslinking precursor that has at least three functional groups of a second type capable of reacting with the first type of functional groups.

*Hydrogels and Precursor Materials*

Suitable hydrogels for use in accordance with the present disclosure include macromolecular and polymeric materials into which water and small hydrophilic molecules can easily diffuse. Hydrogels of interest include, e.g., those prepared through the cross-linking of: polyethers, e.g. polyakylene oxides such as poly(ethylene glycol), poly(ethylene

oxide), poly(ethylene oxide)-co-poly(propylene oxide) block copolymers; poly(vinyl alcohol); and poly(vinyl pyrrolidone). Because of their high degree of biocompatibility and resistance to protein adsorption, polyether derived hydrogels may be useful in some embodiments, including poly(ethylene glycol) derived hydrogels.

Natural polymers, for example proteins, polysaccharides, or glycosaminoglycans, may also be used, as well as derivatives thereof, e.g., hyaluronic acid, dextran, chondroitin sulfate, heparin, heparin sulfate, alginate, gelatin, collagen, albumin, ovalbumin, polyamino acids, collagen, fibrinogen, albumin, fibrin, starch, dermatan sulfate, keratan sulfate, dextran sulfate, pentosan polysulfate, chitosan, fibronectin, laminin, elastin, and active peptide domains thereof. Such polymers may be reacted via functional groups such as amines, thiols, or carboxyls on their amino acids, or derivatized to have activatable functional groups. While natural polymers may be used in low-swelling hydrogels of the present disclosure, their time to gelation and ultimate mechanical properties should be controlled by appropriate introduction of additional functional groups and selection of suitable reaction conditions, e.g., pH. For example, fibrin glues, which rely on polymerization of fibrinogen to form fibrin, have a limited range of mechanical properties, a limited range of degradability, and thus may not be suitable to all of the therapeutic applications that are available when low-swelling hydrogels as described herein are formulated. However, it is contemplated such natural materials may be utilized in some embodiments.

The precursors utilized to form low-swelling hydrogels of the present disclosure may have biocompatible and water soluble core groups. As used herein, water soluble refers to a solubility of at least about 1 g/l in water. This core group may be a water soluble molecule with a minimum of three arms. An arm on a hydrogel precursor refers to a linear chain of chemical groups that connect a crosslinkable functional group to a multifunctional center which initiates the polymerization of the polymeric arms. The combination of this multifunctional center and the attached arms may form the core group. A crosslinkable functional group on a hydrogel precursor arm may include a chemical group that participates in a covalent crosslinking reaction between two hydrogel precursor arms.

In embodiments, the core group may be a water soluble polymer. Examples of such polymers that may be used include, for example: polyethers, for example, polyalkylene oxides such as polyethylene glycol ("PEG"), polyethylene oxide ("PEO"), polyethylene oxide-co-polypropylene oxide ("PPO"), co-polyethylene oxide block or random copolymers; polyvinyl alcohol ("PVA"); poly (vinyl pyrrolidinone) ("PVP"); poly (amino acids); dextran; and proteins, as well as derivatives of the foregoing and combinations of the foregoing.

In other embodiments, multifunctional centers may include polyols which, in embodiments, may possess hydroxyl groups for initiation of monomeric groups that may form the arms of the core that can then be functionalized with crosslinkable groups. Depending on the desired number of arms, the polyol may possess from about 3 to about 12 hydroxyl groups, in embodiments from about 4 to about 10 hydroxyl groups. The polyol may also possess other protected or unprotected functional groups. Suitable polyols include glycerol, mannitol, reducing sugars such as sorbitol, pentaerythritol, and glycerol oligomers including hexaglycerol, as well as derivatives thereof and combinations thereof. As would be readily apparent to one skilled in the art, the number of hydroxyl groups should be equivalent to the number of arms on the multi-armed precursor, i.e., the particular polyol chosen should determine the number of arms on the resultant multifunctional core group. In embodiments, a polymer described above, such as polyethylene glycol, may be formed by initiating the polymerization of ethylene oxide with the polyol, thereby forming arms of a multi-armed precursor that may be further functionalized.

Thus hydrogels can be made from a multi-armed precursor with a first set of functional groups and a low molecular weight precursor having a second set of functional groups. The number of arms on the multi-armed precursor may be from about 3 to about 12, in embodiments from about 5 to about 10.

For example, a multi-armed precursor may have hydrophilic arms, e.g., polyethylene glycol, terminated with N-hydroxy succinimide, with the combined molecular weight of the arms being from about 1,000 to about 40,000; artisans will immediately appreciate that all ranges and values within the explicitly stated bounds are contemplated. In some embodiments, it may be desirable to utilize a multi-armed precursor having six arms or eight arms. The molecular weight of an individual arm of such a precursor may be from about 250 to about 5000, in embodiments from about 1000 to about 3000, in other embodiments from about 1250 to about 2500.

In some embodiments, six-armed or eight-armed precursors may be reacted with a low molecular weight precursor such as trilysine. The trilysine provides multiple points of reaction for crosslinking the multi-armed precursors and it presumably (without being limited to a particular theory of action) allows relatively little movement in terms of shrinking or swelling, with such movement probably being related to the multi-armed precursors, which are relatively larger and more mobile. Accordingly, other small molecules may be used instead of trilysine, for example, molecules with a molecular weight of from about 100 to about 5000, in embodiments from about 300 to about 2500, in other embodiments from about 500 to about 1500. Such small molecules may have at least about three functional groups, in embodiments from about 3 to about 16 functional groups; ordinary artisans will appreciate that all ranges and values between these explicitly articulated values are contemplated. In some cases dilysines and/or tetralysines may be utilized as the low molecular weight precursor.

Such small molecules, also referred to herein as low molecular weight precursors, may be polymers or non-polymers, and may be natural or synthetic. Synthetic refers to a molecule not found in nature and does not include a derivatized version of a natural biomolecule, e.g., collagen with modified side groups. Polyamino acid polymers generated synthetically are normally considered to be synthetic if they are not found in nature and are engineered to not be identical to naturally occurring biomolecules. For instance, trilysine is synthetic since it is not found in nature (even though some bacteria might produce relatively larger polylysines).

In embodiments, a suitable low molecular weight precursor may include a precursor that includes an oligopeptide sequence of no more than about five residues having at least two lysine groups. As used herein, a residue includes an amino acid, either as occurring in nature or derivatized thereof. The backbone of such an oligopeptide may be natural or synthetic. In some embodiments, two or more lysines may be combined with a synthetic backbone to make a precursor; certain embodiments of such precursors may have a molecular weight from about 100 to about 10,000, in embodiments from about 300 to about 5000; artisans will immediately appreciate that all ranges and values between these explicitly articulated bounds are contemplated.

Some hydrogels may be made with a polyethylene glycol-containing precursor. Polyethylene glycol (PEG, also referred to herein as polyethylene oxide) refers to a polymer with a repeat group $(CH_2CH_2O)_n$, with n being at least 3. A polymeric precursor including a polyethylene glycol may thus have at least three of these repeat groups connected to each other in a linear series. The polyethylene glycol content of a polymer or arm may be calculated by adding up all of the polyethylene glycol groups on the polymer or arm, even if they are interrupted by other groups. Thus, an arm having at least 1000 MW polyethylene glycol has enough $CH_2CH_2O$ groups to total at least 1000 MW. As is customary terminology in these arts, a polyethylene glycol polymer does not necessarily terminate in a hydroxyl group.

In certain embodiments, precursors may include macromer compositions that are biodegradable, crosslinkable, and substantially water soluble. The macromers may possess at least one water soluble region, at least one degradable regions, and the arms of such a precursor may possess statistically more than 1 polymerizable region on average, so that a three-armed precursor may possess at least three polymerizable regions. In embodiments, the polymerizable regions may be separated from each other by at least one degradable region. Alternatively, if biodegradability is not desirable, compositions that do not contain the biodegradable segments, but that may be water soluble and crosslink in vivo under physiological acceptable conditions, may be used.

Precursors with longer distances between crosslinks are generally softer, more compliant, and more elastic. Thus, an increased length of a water-soluble segment, such as a polyethylene glycol, may enhance elasticity to produce desirable physical properties in a hydrogel formed from such a precursor. Thus certain embodiments of the present disclosure are directed to precursors with water soluble segments, in embodiments, arms, having molecular weights from about 200 to about 100,000, in embodiments from about 250 to about 35,000, in other embodiments from about 300 to about 5,000.

A monomeric or macromeric precursor capable of being crosslinked to form a biocompatible material may be used to form the hydrogels. These may be small molecules, such as acrylic acid or vinyl caprolactam, larger molecules containing polymerizable groups, such as acrylate-capped polyethylene glycol (PEG-diacrylate), or other polymers containing ethylenically-unsaturated groups, such as those of U.S. Patent No. 4,938,763 to Dunn et al., U.S. Patent Nos. 5,100,992 and 4,826,945 to Cohn et al., or U.S. Patent Nos. 4,741,872 and 5,160,745 to De Luca et al., the entire disclosures of each of which are incorporated by reference herein.

In embodiments, suitable macromeric precursors which may be utilized include the crosslinkable, biodegradable, water-soluble macromers described in U.S. Patent No. 5,410,016 to Hubbell et al., the entire disclosure of which is incorporated by reference herein. These monomers may be characterized by having at least two polymerizable groups, separated by at least one degradable region. When polymerized in water, these monomers may form coherent gels that persist until eliminated by self-degradation. The macromers are self-condensible, meaning that they may react with each other and not with proteins or other moieties on nearby tissues.

*Biodegradable Linkages*

As noted above, in embodiments one or more precursors having biodegradable linkages present in between functional groups may be used to make a hydrogel of the present disclosure biodegradable or absorbable. In some embodiments, these linkages may be, for example, esters, which may be hydrolytically degraded in physiological solution. The use of such linkages is in contrast to protein linkages that may be degraded by proteolytic action. A biodegradable linkage optionally may also form a part of a water soluble core of one or more of the precursors. Alternatively, or in addition, functional groups of precursors may be chosen such that the product of the reaction between them results in a biodegradable linkage. For each approach, biodegradable linkages may be chosen such that the resulting biodegradable biocompatible crosslinked polymer degrades or is absorbed in a desired period of time. Generally, biodegradable linkages may be selected that degrade the hydrogel under physiological conditions into non-toxic or low toxicity products.

The biodegradable linkage may be chemically or enzymatically hydrolyzable or absorbable. Illustrative chemically hydrolyzable biodegradable linkages include polymers, copolymers and oligomers of glycolide, dl-lactide, l-lactide,

caprolactone, dioxanone, and trimethylene carbonate. Other chemically hydrolyzable biodegradable linkages can be monomeric in form, for example those formed through ring opening of glutaric anhydride with poly(ethylene glycol) to form a glutaric acid linkage. Other linkages include succinic, maleic, methyl succinic, diglycolic, methyl glutaric, combinations thereof, and the like. Illustrative enzymatically hydrolyzable biodegradable linkages include peptidic linkages cleavable by metalloproteinases and collagenases. Additional illustrative biodegradable linkages include polymers and copolymers of poly(hydroxy acid)s, poly(orthocarbonate)s, poly(anhydride)s, poly(lactone)s, and poly(phosphonate)s.

Natural polymers may be proteolytically degraded by proteases present in the body, which are enzymes that recognize specific biological moieties such as amino acid sequences. In contrast, synthetic polymers without such specifically cleavable sequences may be degraded by other mechanisms such as hydrolytic degradation. In the spinal cord, synthetic polymers free of such sequences can be expected to undergo little or no degradation by specific enzymatic action. Nonspecific attack and degradation by nonspecifically acting enzymes may result in a different biological response and time to degradation and is not equivalent to degradation by enzymes that are specific to a particular amino acid sequence. Some embodiments of the present disclosure include precursors that do not have sequences subject to specific recognition and cleavage by enzymes.

*Functional Groups*

Functional groups on the precursors include chemical moieties that react with other functional groups to form a covalent bond as part of the process of making a hydrogel. Functional groups include, for example, ethylenically unsaturated polymerizable groups, e.g., pendent vinyl groups, acrylate groups, methacrylate groups, ethacrylate groups, 2-phenyl acrylate groups, acrylamide groups, methacrylamide groups, itaconate groups, styrene groups, combinations thereof, and the like.

Functional groups can also include electrophilic or nucleophilic groups that participate in an electrophilic-nucleophilic reaction to form a hydrogel. Examples of electrophilic functional groups include carbodiimidazole groups, sulfonyl chloride groups, chlorocarbonate groups, n-hydroxysuccinimidyl ester groups, succinimidyl ester groups, sulfasuccinimidyl ester groups, N-hydroxyethoxylated succinimide ester groups, methane diisocyanate groups, methylene-bis(4-cyclohexylisocyanate) groups, isocyanate groups, diisocyanate groups, hexamethylenediisocyanate groups, maleimide groups, and the like. Examples of nucleophilic functional groups include amine groups, hydroxyl groups, carboxyl groups, thiol groups, and the like.

*Initiating Systems*

An initiator group is a chemical group capable of initiating a free radical polymerization reaction. For instance, it may be present as a separate component, or as a pendent group on a precursor. Initiator groups include thermal initiators, photoactivatable initiators, oxidation-reduction (redox) systems, combinations thereof, and the like.

Long wave UV and visible light photoactivatable initiators include, for example, ethyl eosin groups, 2,2-dimethoxy-2-phenyl acetophenone groups, other acetophenone derivatives, thioxanthone groups, benzophenone groups, camphorquinone groups, combinations thereof, and the like.

Examples of thermally reactive initiators include 4,4' azobis(4-cyanopentanoic acid) groups, analogs of benzoyl peroxide groups, combinations thereof, and the like. Several commercially available low temperature free radical initiators, such as V-044, available from Wako Chemicals USA, Inc. (Richmond, VA) may be used to initiate free radical crosslinking reactions at body temperatures to form hydrogels with the aforementioned monomers.

Metal ions may also be used as either an oxidizer or a reductant in redox initiating systems. For example, ferrous ions may be used in combination with a peroxide or hydroperoxide to initiate polymerization, or as parts of a polymerization system. In this case, the ferrous ions would serve as a reductant. Alternatively, metal ions may serve as an oxidant. For example, the ceric ion (4+ valence state of cerium) may interact with various organic groups, including carboxylic acids and urethanes, to remove an electron to the metal ion, thus leaving an initiating radical behind on the organic group. In such a system, the metal ion acts as an oxidizer. Potentially suitable metal ions for either role are any of the transition metal ions, lanthanides and actinides, which have at least two readily accessible oxidation states. In embodiments, metal ions may have at least two states separated by only one difference in charge. Of these, the most commonly used include ferric/ferrous; cupric/cuprous; ceric/cerous; cobaltic/cobaltous; vanadate V vs. IV; permanganate; and manganic/manganous. Peroxygen containing compounds, such as peroxides and hydroperoxides, including hydrogen peroxide, t-butyl hydroperoxide, t-butyl peroxide, benzoyl peroxide, and cumyl peroxide, may also be used.

An example of an initiating system is the combination of a peroxygen compound in one solution, and a reactive ion, such as a transition metal, in another. In this case, no external initiators of polymerization may be needed and polymerization may proceed spontaneously and without application of external energy or use of an external energy source when two complementary reactive functional groups containing moieties interact at the application site.

*Hydrogel Swellability*

It has been found that changing the length of the arms on a precursor while holding other properties generally constant can alter the swelling properties of the resultant gel from one that swells to one that shrinks. At any given concentration of reactive polymer, an arm length can be utilized that provides for a low-swelling gel with minimal compromise of other properties of the hydrogel. Without being bound to a particular theory, changing the arm length can approximate the distance between crosslinks at equilibrium swelling. The closer the arm length is to equilibrium crosslink distance, the less the arms extend in response to swelling.

As described herein, hydrogels may be made in situ in a patient with a low, or even negative, amount of swelling. Such hydrogels may be formulated with mechanical properties for adhesion and/or sealing. In contrast, conventional hydrogels for in situ polymerization that have mechanical properties for adhesion and/or sealing lack low-swelling properties and are not suited for use in a vertebral column, in areas of defined volume, or where minimal swelling is desired.

Thus, desirable hydrogels described herein can include low-swelling hydrogels with a reaction time, density, strength, and desirable medical properties that are made using components selected from a class of precursors in a desirable molecular-weight range, solubility, arm-length, chemical composition, chemical structure, chemical composition, density, precursor concentration, arm number, and with desired functional groups and buffers. Some of these parameters are interrelated so that the choice of one range of starting properties or materials can affect the choice of other properties and materials.

Unless otherwise indicated, swelling of a hydrogel relates to its change in volume (or weight) between the time of its formation when crosslinking is effectively complete and the time after being placed in a physiological solution in an unconstrained state for twenty-four hours, at which point it may be reasonably assumed to have achieved its equilibrium swelling state. For most embodiments, crosslinking is effectively complete within no more than about fifteen minutes, and often within a few seconds, such that the initial weight can be reasonably noted as "Weight at initial formation." Accordingly, the following formula may be used to determine swelling:

$$\% \text{ swelling} = [(\text{Weight at 24 hours} - \text{Weight at initial formation}) / \text{Weight at initial formation}] * 100.$$

Low-swellable or low-swelling hydrogels of the present disclosure may have a weight upon polymerization that increases no more than about 50% by weight upon exposure to a physiological solution, or that shrink (decrease in weight and volume), e.g., by about 5% or more. This is contrary to other hydrogels, which may experience swelling in amounts of from about 300% to about 600% by weight upon exposure to a physiological solution. Embodiments include, for example, hydrogels that have a weight increase from formation to equilibrium hydration of no more than from about 0% to about 50%, in embodiments from about 10% to about 40%, or swell from about 0% to about 50%, in embodiments from about 5% to about 40%, or shrink by a weight decrease of from about 1% to about 50%, in embodiments from about 5% to about 30%. Again, swelling or shrinking is determined by the change in weight of the hydrogel upon exposure to a physiological solution utilizing the formula set forth above.

In some embodiments, shrinkage may be referred to herein as a negative % swelling; thus, in embodiments, a hydrogel of the present disclosure may swell from about -50% to about 50%, in other embodiments a hydrogel may swell from about -20% to about 40%. Artisans will immediately appreciate that all ranges and values within or otherwise relating to these explicitly articulated limits are disclosed herein.

The weight of the hydrogel includes the weight of the solution in the hydrogel. A hydrogel formed in a location wherein it is constrained is not necessarily a low-swelling hydrogel. For instance, a swellable hydrogel created in a body may be constrained from swelling by its surroundings but nonetheless may be a highly swellable hydrogel as evidenced by measurements of its swelling when unconstrained and/or the forces against a constraint.

The solids content of the hydrogel which has crosslinked and is at equilibrium can affect its mechanical properties and biocompatibility and reflects a balance between competing requirements. In general, a relatively low solids content may be desirable, e.g., from about 5% to about 25% of the combined weight of the hydrogel in an aqueous solution, in embodiments all ranges and values therebetween, e.g., from about 5% to about 10%, from about 10% to about 15%, from about 5% to about 15%, and less than about 15%, or less than about 20%.

*In situ Polymerization*

Formulations may be prepared that are suited to make precursor crosslinking reactions occur "in situ", meaning

they occur at a tissue in a living animal or human body. In general, this may be accomplished by having a precursor that can be activated at the time of application to a tissue to form a crosslinked hydrogel. Activation can be made before, during, or after application of the precursor to the tissue, provided that the precursor is allowed to conform to the tissue's shape before crosslinking and associated gelation is otherwise too far advanced. Activation includes, for example, triggering a polymerization process, initiating a free radical polymerization, or mixing precursors with functional groups that react with each other. Thus, in situ polymerization may include activation of chemical moieties to form covalent bonds to create an insoluble material, e.g., a hydrogel, at a location where the material is to be placed on, within, or both on and within, a patient. In situ polymerizable polymers may be prepared from precursors that can be reacted such that they form a polymer within the patient. Thus, precursors with electrophilic functional groups can be mixed or otherwise activated in the presence of precursors with nucleophilic functional groups. In other embodiments, precursors with ethylenically unsaturated groups can be initiated to polymerize in situ on the tissue of a patient.

Certain functional groups, such as alcohols or carboxylic acids, do not normally react with other functional groups, such as amines, under physiological conditions (e.g., pH 7.2, 37°C). However, such functional groups can be made more reactive by using an activating group such as N-hydroxysuccinimide. Suitable activating groups include carbonyldiimidazole, sulfonyl chloride, aryl halides, sulfosuccinimidyl esters, N-hydroxysuccinimidyl ester, succinimidyl ester, epoxide, aldehyde, maleimides, imidoesters and the like. The N-hydroxysuccinimide esters or N-hydroxysulfosuccinimide groups may be groups of particular interest for crosslinking of proteins or amine functionalized polymers such as amino terminated polyethylene glycols.

Hydrogels may be formed either through covalent, ionic or hydrophobic bonds introduced through, e.g., chemical cross-linking agents or electromagnetic radiation, such as ultraviolet light, of both natural and synthetic hydrophilic polymers, including homo and co-polymers. Physical (non-covalent) crosslinks may result from, e.g., complexation, hydrogen bonding, desolvation, Van der Waals interactions, or ionic bonding, and may be initiated by mixing components that are physically separated until combined in situ, or as a consequence of a prevalent condition in the physiological environment, such as temperature, pH, and/or ionic strength. Covalent crosslinking may be accomplished by any of a number of mechanisms, including free radical polymerization, condensation polymerization, anionic or cationic polymerization, step growth polymerization, and electrophile-nucleophile reactions.

In some embodiments, hydrogel systems may include those biocompatible multicomponent systems that spontaneously crosslink when the components are mixed, but wherein the two or more components are individually stable for the duration of the deposition process. Such systems include, for example, a first component including macromers that are di- or multifunctional amines, and a second component including di- or multifunctional oxirane containing moieties. Other initiator systems, such as components of redox type initiators, may also be used.

In addition, hydrogels formed in accordance with the present disclosure may be used as coatings. Such coatings may be formed as laminates (i.e., having multiple layers). Thus, for example, a lower layer of the laminate may possess a more tightly crosslinked hydrogel that provides good adherence to the tissue surface and serves as a substrate for an overlying compliant coating to reactively bond thereto. Materials having lower molecular weights between crosslinks may be suitable for use as a base coating layer. Molecular weights from about 400 to about 20,000 of polyethylene glycol may be useful for such applications, with molecular weights from about 500 to about 10,000 utilized in some embodiments.

Some embodiments of forming a hydrogel involve mixing precursors that crosslink quickly after application to a surface, e.g., on a tissue of a patient, to form a biodegradable hydrogel. With respect to coating a tissue, and without limiting the present disclosure to a particular theory of operation, it is believed that reactive precursor species that crosslink quickly after contacting a tissue surface may form a three dimensional structure that is mechanically interlocked with the coated tissue. This interlocking contributes to adherence, intimate contact, and continuous coverage of the coated region of the tissue. The crosslinking reaction leading to gelation can occur, in some embodiments, within a time from about 1 seconds to about 5 minutes, in embodiments from about 3 seconds to about 1 minute; persons of ordinary skill in these arts will immediately appreciate that all ranges and values within these explicitly stated ranges are contemplated. In some cases gelation may occur in less than about 10 seconds.

The precursors may be placed into solution prior to use, with the solution being delivered to the patient. The hydrogel system solutions should not contain harmful or toxic solvents. In embodiments, the precursors may be substantially soluble in water to allow application in a physiologically-compatible solution, such as buffered isotonic saline. One may use a dual syringe or similar device to apply the precursor solutions, including those described in U.S. Patent Nos. 4,874,368; 4,631,055; 4,735,616; 4,359,049; 4,978,336; 5,116,315; 4,902,281; 4,932,942; 6,179,862 ; 6,673,093; and 6,152,943. Further, such precursors may be used in combination with visualization agents such as a dye. Suitable dyes are within the purview of those skilled in the art and may include, for example, a dye for visualizing a thickness of the hydrogel as it is formed in situ, e.g., as described in U.S. Patent No. 7,009,034, the entire disclosure of which is incorporated by reference herein. In some embodiments, a suitable dye may include FD&C Blue #1, FD&C Blue #2, FD&C Blue #3, D&C Green #6, methylene blue, combinations thereof, and the like.

Embodiments of hydrogels described herein include low-swelling, in-situ formed, precursor based medical

crosslinked hydrogels, which optionally possess a gelation time in situ of less than about twenty seconds (or less than about ten seconds, or less than about five seconds). Such hydrogels may be made with precursors having a solubility of from about 1 gram per liter to at least about 10 grams per liter. Such hydrogels may be prepared with a 1:1 ratio of reactive functional groups (e.g., electrophile: nucleophile) or other ratios as suited to the formulation. Buffers may be used to provide a pH for maintaining the activity of a reactive functional group in solution ("pot life") and to provide a desired osmotic balance when mixed, e.g., a physiological range as described in U.S. Patent No. 7,009,034. The arms may have a terminal functional group, or a functional group, e.g., within no more than from about 10,000 to about 5,000 MW of the free end of an arm. At least one functional group, more than one functional group, or a combination thereof may be present. The number of arms for at least one precursor of the low-swelling hydrogel may be from about 3 to about 12, in embodiments from about 4 to about 8.

An example of an 8-armed precursor, having PEG arms functionalized with succinimidyl glutarate, includes, for example, the following:

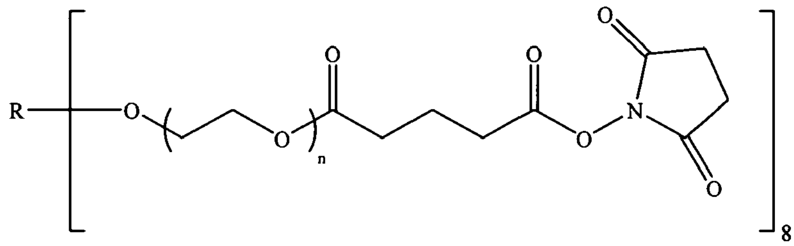

wherein R is a core as described above, in embodiments a hexaglycerin core, and n may be from about 4 to about 150, in embodiments from about 10 to 100. In embodiments, the total molecular weight of the 8 arms may be about 20,000. In other embodiments, PEG arms functionalized with succinimidyl succinate may be utilized.

In accordance with the present disclosure, and as set forth in greater detail in the Examples below, it has been found that increasing the number of arms on the precursor, and/or decreasing the arm length of the arms on the precursor, may result in an increase in crosslink density (# of crosslinks/gram of gel). As the crosslink density increases, the equilibrium swelling may decrease. Thus, it is possible to change the swelling characteristics of a hydrogel by altering its crosslinking density.

Therefore, in accordance with the present disclosure, one may be able to tailor the degree of swelling or shrinking of a composition of the present disclosure depending upon its intended use, by selecting the appropriate number of arms and arm length, i.e., the combined molecular weight of the arms. One can shorten the arms, or increase the number of arms on the reactive PEG to achieve this result. Another factor is the concentration of the PEG precursors, for example, in solution.

As noted above, the number of arms on a precursor may vary depending on the desired degree of swelling and/or shrinking. In embodiments, 4 arm, 6 arm, and/or 8 arm precursors may be utilized. As noted above, the extent of swelling or shrinking may also be controlled by the number of arms and the combined weight (which correlates to the length) of the arms. The combined weight of the arms may be from about 750 to about 20000, in embodiments from about 5000 to about 18000, in other embodiments from about 10000 to about 17500, in other embodiments from about 12000 to about 15000. The arm length and the concentration of PEG precursors may, in embodiments, determine how close a gel is to equilibrium swelling when it is formed.

*Applications at the Vertebral Column*

Nerves near the vertebral column may be vulnerable to compression in response to tissue inflammation or swelling of materials surgically placed into the body. While the body can normally tolerate a certain amount of swelling of implanted materials, swelling near a bone or rigid implant may be less tolerated because forces may be directed away from bone towards sensitive soft tissue. It thus may be desirable to avoid compressing a nerve in this manner.

*Tissue Augmentation Applications*

Low swelling hydrogels of the present disclosure may also be suitable for use in cosmetic surgery, for example in wrinkle filling, in sphincter augmentation applications, in treatments of carpal tunnel injuries and disorders, including carpal tunnel syndrome, and the like. Other suitable uses for low swelling hydrogels of the present disclosure include sealants in the eye, anastomotic sealants, and/or sealants for prostate surgery.

Regardless of the application, changes in the mechanical properties of the low swelling hydrogels of the present

disclosure over time are related to degradation of the hydrogel, and not to any changes in dimension.

*Methods of Using Biocompatible Polymers*

As noted above, in other embodiments an application for a low-swelling hydrogel of the present disclosure may be for use in or around a vertebral column. The low-swelling nature of the hydrogel minimizes compression of tissues, especially nerves, against the bone. The hydrogel may be applied exterior to the theca, which is the dura mater of the spinal cord. In some applications, the hydrogel may be applied substantially exterior to a theca in the vertebral column, meaning that the hydrogel is applied in the vertebral column even while the theca is damaged or even breached, but excluding situations wherein the spinal cord is essentially severed and the hydrogel is placed into the nerve gap. The hydrogel of the present disclosure may thus be placed onto tissue adjacent a nerve or in a space between a nerve and such surrounding tissue. The hydrogel may also contact associated vertebral-column structures, and fill some or all of the vertebral foramen, and regions outside, including nerve roots and nerve portions exterior to the theca and within, e.g., from about 0.1 cm to about 5 cm of the vertebral column, in embodiments from about 1 cm to about 4 cm of the vertebral column. As such, the hydrogel may function as a tissue adhesive, tissue sealant, drug delivery vehicle, wound covering agent, barrier to prevent postoperative adhesions, or a covering of inflamed or injured sites. The hydrogel may be applied as a bolus that fills a void or lumen and/or as a coating that conforms to a tissue surface.

In embodiments, as noted above, hydrogels of the present disclosure may also be utilized in cosmetic surgery. For example, bulking of skin tissues, including fascia, subcutaneous and dermal tissues, may be used to treat skin disorders including scars, skin laxness, and skin thinning, and may be used in some types of cosmetic and reconstructive plastic surgery. Such disorders of the skin often are exhibited as contour deficiencies, which may be treated using the hydrogels of the present disclosure. Contour deficiencies in the skin can occur as a result of factors such as aging, environmental exposures, weight loss, childbearing, surgery or disease. Contour deficiencies include frown lines, worry lines, wrinkles, crow's feet, marionette lines, stretch marks, internal and external scars, combinations thereof, and the like. Augmentation of the skin layers with hydrogels of the present disclosure may thus reduce or eliminate such contour deficiencies.

The hydrogels may be injected into the desired skin layer, without having to worry about over-swelling which may distend tissue. As a wrinkle filler, the low swelling hydrogels of the present disclosure can be injected or otherwise placed subcutaneously in a liquid form, with gelling occurring after administration. The low swelling hydrogels of the present disclosure can advantageously be shaped or spread thinly to achieve the desired effect while still in a liquid form. Similarly, for cosmetic or reconstructive surgery applications, the low swelling hydrogels of the present disclosure can be applied to a selected area of the body in a liquid form (or can be formed prior to insertion as described herein), and can be manipulated into the desired shape or to fill a desired volume. Reconstructive surgery or aesthetic enhancement may incorporate the low swelling hydrogels of the present disclosure. Regions of the face, such as cheeks, nose, ears, and skin adjacent the eyes (soft tissue) can be reconstructively augmented or enhanced using the low swelling hydrogels of the present disclosure.

Moreover, low swelling hydrogels of the present disclosure may be utilized in sphincter augmentation applications including, but not limited to, urinary (urethral), anal, and esophageal sphincter augmentation. Any method within the purview of those skilled in the art may be utilized to introduce a low swelling hydrogel of the present disclosure into a sphincter. As would be apparent to one skilled in the art, the method selected may depend, in part, upon the location of the sphincter within the body.

For example, a low swelling hydrogel of the present disclosure may be delivered to a target tissue site to augment a mammalian sphincter, such as the lower esophageal sphincter (LES). In embodiments, a catheter assembly may be utilized to introduce the compositions of the present disclosure. Such catheter assemblies may include a flexible catheter having a distal end affixed to an injection needle may be utilized to introduce a low swelling hydrogel of the present disclosure into the sphincter. The catheter may be coupled to a syringe at its proximal end. The syringe may contain the low swelling hydrogel of the present disclosure by a standard luer connection. The needle may pierce tissue at or adjacent the sphincter to deliver a low swelling hydrogel of the present disclosure to a portion of the sphincter. Pressure may then be applied to the syringe plunger, which then injects the low swelling hydrogel of the present disclosure into the lumen of the catheter and, subsequently, the needle.

In embodiments, the catheter and needle may be guided to the treatment site by a means to assist visualization in vivo, for example an endoscope having a steering and visualization means.

The first several layers of sphincter include a mucosal layer, a submucosal layer and an underlying smooth muscle layer. The needle may be positioned to produce controlled tissue bulking/augmentation in the smooth muscle layer underlying the mucosal and submucosal layers. In embodiments, the needle may be positioned to inject controlled amounts of low swelling hydrogel of the present disclosure in the portion of smooth muscle tissue that lies from about 1 mm to about 4 mm from the surface of the mucosal layer.

A similar approach may also be used to correct other sphincter deficiencies. For example, the urethral sphincter

may be augmented to alleviate incontinence. Similarly, the pyloric sphincter may also be augmented to reduce "dumping" problems associated with intestinal pH imbalance.

In yet other embodiments, low swelling hydrogels of the present disclosure may be utilized in treatments of carpal tunnel injuries and disorders, including carpal tunnel syndrome. Such treatments may include, in embodiments, encapsulation of the carpal tunnel. For example, in embodiments, a low swelling hydrogel of the present disclosure may be introduced into the carpal tunnel by injection or otherwise placed in the carpal tunnel in liquid form, and allowed to gel, thereby encapsulating the carpal tunnel and forming a barrier between the surface of the carpal tunnel and tendons and nerves therein, including the median nerve, thereby reducing inflammation and/or irritation.

The hydrogels of the present disclosure may also be used for drug delivery. Biologically active agents or drug compounds that may be added and delivered from the crosslinked polymer or gel include, for example: proteins, glycosaminoglycans, carbohydrates, nucleic acids, inorganic and organic biologically active compounds. Specific biologically active agents include, but are not limited to: enzymes, antibiotics, antimicrobials, antineoplastic agents, local anesthetics, hormones, angiogenic agents, anti-angiogenic agents, growth factors, antibodies, neurotransmitters, psychoactive drugs, anticancer drugs, chemotherapeutic drugs, drugs affecting reproductive organs, genes, anti-inflammatory drugs, analgesics, antibiotics, anti-proliferatives, anti-fibrotics, and oligonucleotides.

The bioactive compounds described above may be mixed with a precursor prior to making the aqueous solution or during the aseptic manufacturing of the precursor. This mixture may then be mixed with another precursor to produce a crosslinked material in which the biologically active substance is entrapped. Precursors made from inert polymers like PLURONICS®, TETRONICS®, or TWEEN® surfactants may be used, for example, with small molecule hydrophobic drugs.

In some embodiments, the active agent or agents may be present in a separate phase when precursors are reacted to produce a crosslinked polymer network or gel. This phase separation may prevent participation of bioactive substances in a chemical crosslinking reaction. The separate phase may also help to modulate the release kinetics of active agent from the crosslinked material or gel, where 'separate phase' could be an oil (for example, an oil-in water emulsion), a biodegradable vehicle, and the like.

In order that those skilled in the art may be better able to practice the features of the present disclosure described herein, the following examples are provided to illustrate, but not limit, the features of the present disclosure.

EXAMPLES

Example 1: Low swelling hydrogel formulations

To prepare the hydrogels, trilysine with primary amine functional groups was reacted with multiarmed polyethylene glycol (PEG) electrophilic precursors with succinimidyl ester electrophilic functional groups (specifically, succinimidyl glutarate, SG) on the end of each of four arms (4a) having a total MW of about 20,000 MW polyethylene glycol (sometimes referred to herein as 4a20k SG) in a 1:1 stoichiometric ratio of electrophiles: nucleophiles.

Essentially identical hydrogels were then made, with the ratios of the electrophilic-nucleophilic functional groups still being 1:1, except that a 6-armed (6a) or 8-armed (8a) precursor (with functional groups on the end of each arm) with PEG arms having a total MW of about 10,000 (10k) or 20,000 (20k) were used instead of the four-armed precursor. (Thus, the other hydrogels included 6a10K SG, 6a20K SG, 8a10K SG, and 8a20K SG.)

A detailed procedure for making a hydrogel is as follows, using 4a20k SG as an example. Trilysine was mixed into a 0.075 M Borate buffer at a concentration of 0.005 mg/ml. The resultant pH of the solution was approximately 10. 4a20k SG was reconstituted at 0.2 g/ml with a weak phosphate buffer at pH 4. The two liquid components were combined by forcing them through a static mixer into silicone tubing. The tubing was cut into disks and the gel was removed. Individual disks were weighed and placed into PBS at 37° C. After 24 hours the disks were weighed again and % swelling was calculated. Gel time was measured by injecting one component into a test tube containing the second component and a stir bar. A stopwatch was started at the time of injection and stopped when the stir bar exhibited a perceptible change in speed. The gels formed in the gel time measurement were used to determine persistence time. Individual gel plugs were placed in phosphate buffered saline at 37° C and monitored daily until they were not visible to the naked eye.

Other formulations were similarly made, with varying concentrations and pH: 8a15k SG (8 arm PEG, with the arms having a total combined MW of 15,000, terminated with succinimidyl glutarate) with 0.19 g PEG/ml phosphate, 0.012 g Trilysine/ml borate pH 10; 4a10k SS (4 arm PEG, with the arms having a total combined MW of 10,000, terminated with succinimidyl succinate (SS)) with 0.19 g PEG/ml phosphate, and 0.008 g Trilysine/ml borate at a pH of 10.

Table 1 shows the results obtained for these low swelling hydrogel formulations. Hydrogels prepared with ~ 9% solids and individual arm lengths less than about 2500 MW exhibited low swelling, compared to a hydrogel prepared from a precursor with individual arm lengths of about 5000, with other parameters being held essentially constant.

Table 1

| Formulation | Individual Arm Length /MW | Gel time ± std dev, s | Swelling ± std dev, % | Disappearance, days | Burst Strength, psi |
|---|---|---|---|---|---|
| 8a10k SG[i] | 1250 | 1.3 ± 0.04 | -32.7 ± 5.22 | 60 | 72 ± 11 |
| 6a10kSG[i] | 1667 | 1.5 ± 0.03 | -27.2 ± 2.54 | 60 | |
| 8a20k SG[i] | 2500 | 1.6 ± 0.11 | 12.3 ± 2.18 | 60 | |
| 4a20k SG[ii] | 5000 | 1.2 | 80 | 40 | 93 ± 36 |
| *i, n=3; ii, average based on multiple tests performed separately* | | | | | |

The materials shown in Table 1 were synthesized and tested to verify substitution levels over 95 %. Formulations were balanced at a 1:1 stoichiometry and pH was adjusted give similar gel times. All hydrogels had a gelation time of less than 5 seconds.

The 4 arm hydrogel swelled about 80% by weight (Table 1, 4a20k SG, with 4a indicating 4 arms, 20k indicating 20,000 MW PEG total for the arms, and SG indicating that each arm was terminated with succinimidyl glutarate). The 6 arm and 8 arm gels swelled only about 12% by weight or shrunk by about 27% or about 32% (Table 1).

Disappearance times were measured for the hydrogels (Table 1) by observing the gels in a clear plastic test tube and noting the time at which they were no longer visible to the naked eye, indicating that they were fully degraded. Burst strength was measured and found to be within acceptable ranges.

Example 2: Role of osmotic environment in swelling

The role of osmotic environment in swelling was tested by making a hydrogel using 4a20k SG as described in Example 1 and exposing it to a physiological buffered saline having a pH of 7.0-7.4 and an osmolarity of about 300 mOs or to a double-strength solution of the same saline. With n=3 (hydrogel plugs for each molarity of PBS), the swelling from gelation to equilibrium swelling (taken at 24 hours) averaged 68% for the physiological saline and 57% for double-strength saline. These results indicate that osmolarity differences inherent to the Swelling environment did not account for the reduced swelling of hydrogels formulated with precursors having different arm lengths because the changes in swelling were too small to account for the larger changes generally observed when the length of the arms was increased.

Example 3: Low-swelling hydrogels tested in vivo

Low-swelling hydrogels were implanted in the vertebral column in vivo. Formulation 1 was a hydrogel made from reacting a trilysine precursor reacted with 8a15k SG (8 arm PEG precursor with succinimidyl glutamate on the end of each arm having a total PEG MW of about 15,000), with conditions effectively as described in Example 1 and Table 1. Precursors were applied using dual lumen applicators that mix the solutions and direct them to the site of application.

A total of 15 canines received full width laminectomies at both L2 and L5, after which a 1 cm midline durotomy was created, which was sutured closed. Animals were randomized to remain as controls (n=5 animals; no additional treatment prior to closure), or to receive formulation 1 application at both laminectomy sites using either a DUOFLO® dual lumen applicator (Hemaedics Inc., Malibu, CA) (n=5 animals) or a MICROMYST™ dual lumen applicator (Confluent Surgical, Inc., Waltham, MA) (n=5 animals). Formulation 1 was observed to be adherent to the tissue within a few seconds of its application.

The surgeries were preformed with a single midline skin incision (typically 15 cm length) made in all animals to gain access to both L2 and L5. Laminectomies (average 2.5 cm length, 1.3 cm width) were performed using standard or Kerrison rongeurs. All durotomies were midline and 1 cm in length, and all leaked CSF spontaneously following suturing.

Animals randomized to control had CSF weeping from suture holes at closure. All control sites (10/10) continued to weep CSF from the durotomy needle holes at the time of muscle and fascia closure.

All of the control animals developed postoperative subcutaneous fluid accumulations (5/5, 100%) within 1 to 3 days. All accumulations were contained by the sutured skin closure, and were present at the 1 week exam. Accumulations were presumed to be CSF, and were absorbed with flat incisions at the 4 week exam. Only 1 (10%) of the Formulation 1 animals exhibited postoperative subcutaneous fluid accumulation rate, compared to 5/5 (100%) of the controls. (Formula 1 applied with DUOFLO® had zero leaks, Formula 1 with MICROMYST™ had one leak, all controls leaked.) While not wishing to be bound by any theory the leak of Formula 1 was probably due to applicator (thickness of layer) and not formulation.

Animals randomized to receive Formulation 1 treatment underwent Valsalva's Maneuver to 20 cm $H_2O$ following application. No sites treated with Formulation 1 (20/20) had leakage following hydrogel application, despite the Valsalva's Maneuver.

The average volume applied and thickness over the suture line for the MICROMYST™ group was 1.3 ml volume and 2.7 mm thickness, with 2.2 ml volume and 3.3 mm and thickness for the DUOFLO® group. Since the laminectomy width was the full width of the dural sac, the gutters in each laminectomy site were deep and extended down to the nerve roots. Therefore, while the average Formulation 1 thickness over the sutures was about 3.3 mm, the thickness due to runoff in the gutters was probably approaching 8-10 mm in some cases.

All animals were evaluated for neurological deficits at 1, 4, 8 and 16 weeks. Assessments focused on neurological sequelae for alertness, motor function, cranial nerve function and posture. No neurological deficits were noted in any of the animals enrolled. With the exception of one early death for causes unrelated to the surgeries, all animals remained healthy with no detectable sequelae from the initial surgical procedure. These results indicate that low-swelling hydrogels were effective as applied to the tissue inside the vertebral column and substantially exterior to a theca in the vertebral column, including peridural and epidural spaces and spinal nerves or nerve roots near the vertebral column.

Example 4: Low-swelling hydrogel data

Additional testing of the above samples and additional samples was conducted. Two different variables were evaluated: PEG arm number and PEG arm length. The samples were the 8a20K SG from Example 1, 8a10K SG from Example 1, 4a10K SS from Example 1, and the 4a20K SG from Example 1. Additional samples including a 4a10K SG (a 4 arm PEG, with arms having a total combined weight of about 10000, terminated with succinimidyl glutarate) and a 6a15K SG (a 6 arm PEG, with arms having a total combined weight of about 15000, terminated with succinimidyl glutarate) were prepared utilizing the procedures described above in Example 1. The crosslinking agent for all samples was trilysine, with the ratio of reactive groups (NHS and $NH_2$) at about 1:1. The resulting hydrogels were tested for gel time, swelling (%), and time for disappearance. The results are summarized below in Table 2.

Table 2

| Formulation | Gel time @t=0 (seconds) | Swelling (%) | Disappearance |
|---|---|---|---|
| **8a10K SG** | | | |
| Sample 1 | 1.28 | -37.3692 | 60 Days |
| Sample 2 | 1.28 | -27.0672 | |
| Sample 3 | 1.35 | -33.7097 | |
| Average | 1.303333 | -32.7097 | |
| Standard Deviation | 0.040415 | 5.220888 | |
| **6a15K SG** | | | |
| Sample 1 | 1.53 | -24.2803 | 60 Days |
| Sample 2 | 1.57 | -28.5875 | |
| Sample 3 | 1.5 | -28.7761 | |
| Average | 1.533333 | -27.2146 | |
| Standard Deviation | 0.035119 | 2.542974 | |
| **8a20K SG** | | | |
| Sample 1 | 1.65 | 12.31121 | 60 Days |
| Sample 2 | 1.59 | 10.15413 | |
| Sample 3 | 1.44 | 14.50754 | |
| Average | 1.56 | 12.32429 | |
| Standard Deviation | 0.108167 | 2.176738 | |
| **4a20K SG** (DURASEAL) | ~1.2 | ~80 | ~40 days |

As can be seen from Table 2 above, increasing the number of arms, or decreasing the arm length, both resulted in an increase in crosslink density (# crosslinks/gram of gel). As the crosslink density increased, the equilibrium swelling decreased. The formulations with a high degree of crosslinking lasted about 33% longer than the 4a20K SG (DURASEAL). The swelling data is also summarized in Figure 1. As can be seen in Figure 1, those compositions having combined arm weight of only about 10K shrunk, while those compositions having an increase in the number of arms (8) had much less swelling than those having a lower number of arms (4).

In order to evaluate the mechanical properties of the gels as a function of crosslink density, an Instron Universal Testing Machine was utilized to measure the % strain to failure. Briefly, cylindrical gel plugs were compressed at a constant rate to failure.

The results of compressive strength testing are summarized in Figures 1 and 2. As can be seen in Figure 1, materials with higher degrees of crosslinking reached brittle failure earlier than DuraSeal. This would be expected as the crosslink density should stiffen the hydrogel. The stiffness seemed to follow the following trend: 8a10K > 8a20K = 4a10K > 4a20K.

In order to evaluate how the above stiffness might affect burst strength, several samples were evaluated using a burst strength fixture. Precursors were sprayed over a defect in porcine collagen to form a hydrogel of defined thickness. The sample was pressurized with phosphate buffered saline (PBS) from below the defect until the hydrogel failed. The maximum pressure was recorded on a digital readout attached to a transducer. The results are summarized in Table 3 below.

Table 3

| Formulation | Burst Strength (in water) |
|---|---|
| **8a10K SG** | |
| Sample 1 | 68 |
| Sample 2 | 79 |
| Sample 3 | 77 |
| Sample 4 | 80 |
| Sample 5 | 55 |
| Average | 71.8 |
| Standard Deviation | 10.52141 |
| **4a10K SS** | |
| Sample 1 | 122 |
| Sample 2 | 112 |
| Sample 3 | 85 |
| Sample 4 | 139 |
| Sample 5 | 109 |
| Average | 113.4 |
| Standard Deviation | 19.73069 |
| **4a20K SG** (DURASEAL) | |
| Sample 1 | 70 |
| Sample 2 | 151 |
| Sample 3 | 104 |
| Sample 4 | 66 |
| Sample 5 | 73 |
| Average | 92.8 |
| Standard Deviation | 35.85666 |

As can be seen from the above table, the change in arm length did not have much of an effect on burst strength. However, the most brittle formulation (8a10KSG) had the lowest burst strength.

From the above, one can see that it is possible to change the swelling characteristics of a hydrogel by altering its crosslinking density. One can shorten the arms, or increase the number of arms on the reactive PEG to achieve this result, i.e., stiffening the gel and possibly decreasing burst strength.

A gel between the 8a10K and 8a20K may have zero swelling without significantly compromising the burst strength.

All patent applications, publications, and patents mentioned herein are hereby incorporated by reference herein to the extent that they do not contradict the explicit disclosure of this specification. It will be understood that various modifications may be made to the embodiments disclosed herein. Therefore the above description should not be construed as limiting, but merely as exemplifications of preferred embodiments. Those skilled in the art may envision other modifications within the scope and spirit of the claims appended hereto.

## Claims

**1.** The use of a first synthetic precursor possessing first functional groups in the preparation of a medicament for use in a method of treating tissue which comprises the steps of:

contacting tissue with the first precursor and with a second synthetic precursor comprising a multi-armed precursor possessing a core possessing from about 3 to about 12 arms, the arms each comprising a polyethylene glycol having a molecular weight from about 250 to about 5000 and possessing second functional groups at the ends thereof,
wherein the first functional groups crosslink with the second functional groups thereby forming a hydrogel which swells from about -50% to about 50%.

**2.** The use of claim 1, wherein the hydrogel is crosslinked to form a gel in less than about 10 seconds after contacting the first precursor with the second precursor and/or the method of claim 1, wherein the first functional groups comprise nucleophiles and the second functional groups comprise electrophiles and/or the method of claim 1, wherein the first synthetic precursor is selected from the group consisting of dilysines, trilysines, and tetralysines.

**3.** The use of claim 1 or claim 2, wherein the first synthetic precursor comprises an oligopeptide sequence of no more than about five residues comprising at least two lysine groups.

**4.** The use of any preceding claim, wherein the core of the second precursor is selected from the group consisting of polyethers, polyamino acids, proteins, and polyols; preferably wherein the core of the second precursor is selected from the group consisting of polyethylene glycol, polyethylene oxide, polyethylene oxide-co-polypropylene oxide, co-polyethylene oxide copolymers, polyvinyl alcohol, polyvinyl pyrrolidinone, poly(amino acids), dextran, proteins, derivatives thereof, and combinations thereof.

**5.** The use of any preceding claim, wherein the multi-armed precursor possesses from about 4 to about 8 arms.

**6.** The use of any preceding claim, wherein the combined weight of the arms of the multi-armed precursor is from about 750 to about 20000.

**7.** The use of any of claims 1 to 5, wherein the combined weight of the arms of the multi-armed precursor is from about 5000 to about 18000.

**8.** The use of any preceding claim, further comprising administering a bioactive agent with the first precursor and second precursor.

**9.** The use of any preceding claim, further comprising administering a visualization agent with the first precursor and second precursor; preferably wherein the visualization agent comprises a dye selected from the group consisting of FD&C Blue #1, FD&C Blue #2, FD&C Blue #3, D&C Green #6, methylene blue, and combinations thereof.

**10.** The use of any preceding claim, wherein the hydrogel shrinks by a weight decrease of from about 1% to about 50% or the method of claim 1, wherein the hydrogel shrinks by a weight decrease of from about 5% to about 30%.

11. The use of any preceding claim, wherein the tissue comprises contour deficiencies of skin.

12. The use of claim 11, wherein the contour deficiencies in the skin are found on skin located on a portion of the body selected from the group consisting of cheeks, nose, ears, and skin adjacent an eye; preferably wherein the contour deficiencies in the skin are selected from the group consisting of frown lines, worry lines, wrinkles, crow's feet, marionette lines, stretch marks, internal scars, external scars, and combinations thereof.

13. The use of any preceding claim, wherein the tissue comprises a nerve, tissue adjacent a nerve, or a space between a nerve and adjacent tissue.

14. The use of claim 13, wherein the space adjacent the nerve comprises the interior of the carpal tunnel.

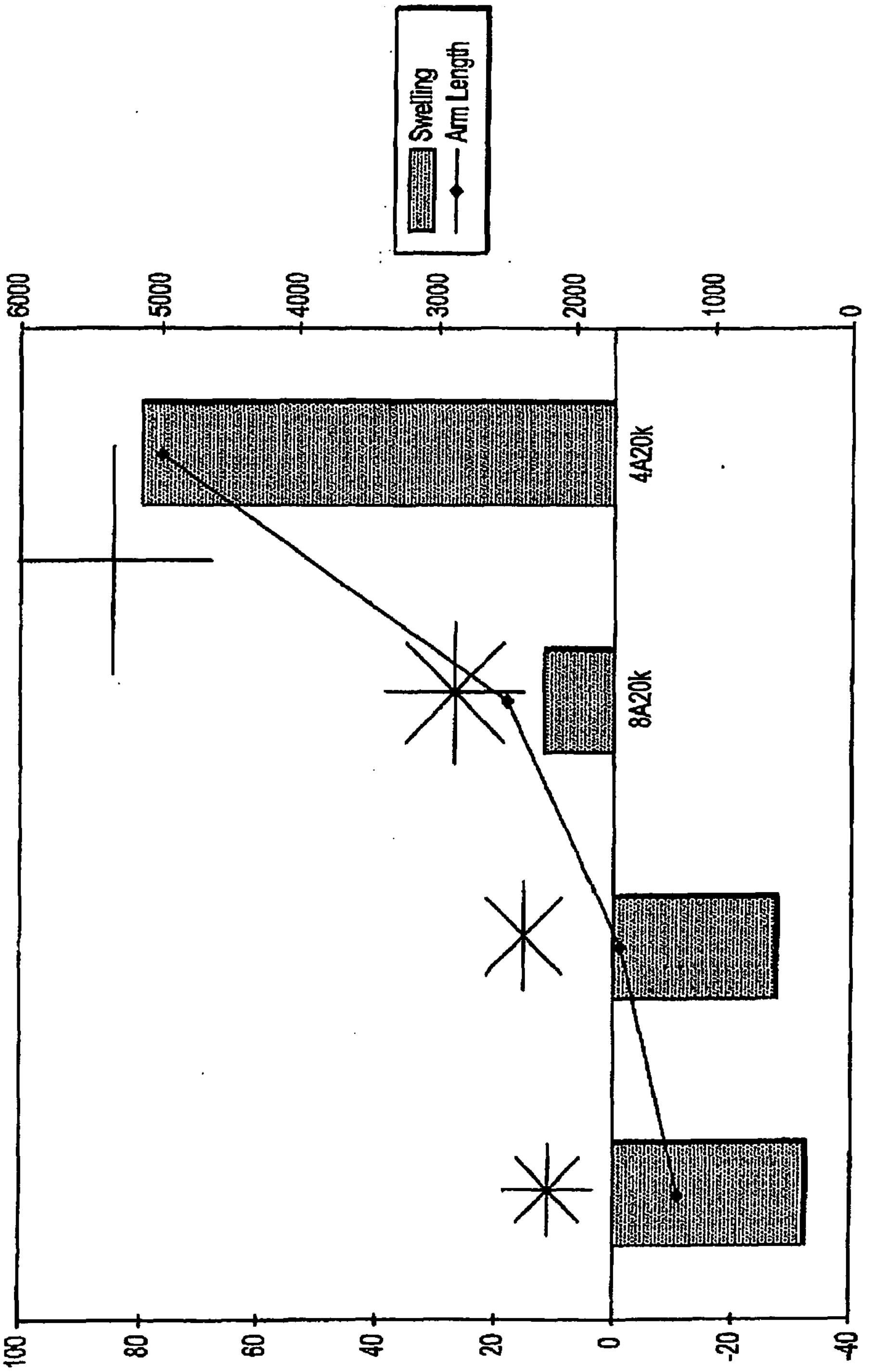
Swelling
Arm Length
6000
5000
4000
3000
2000
1000
0
4A20k
8A20k
100
80
60
40
20
0
-20
-40

**FIG. 1**

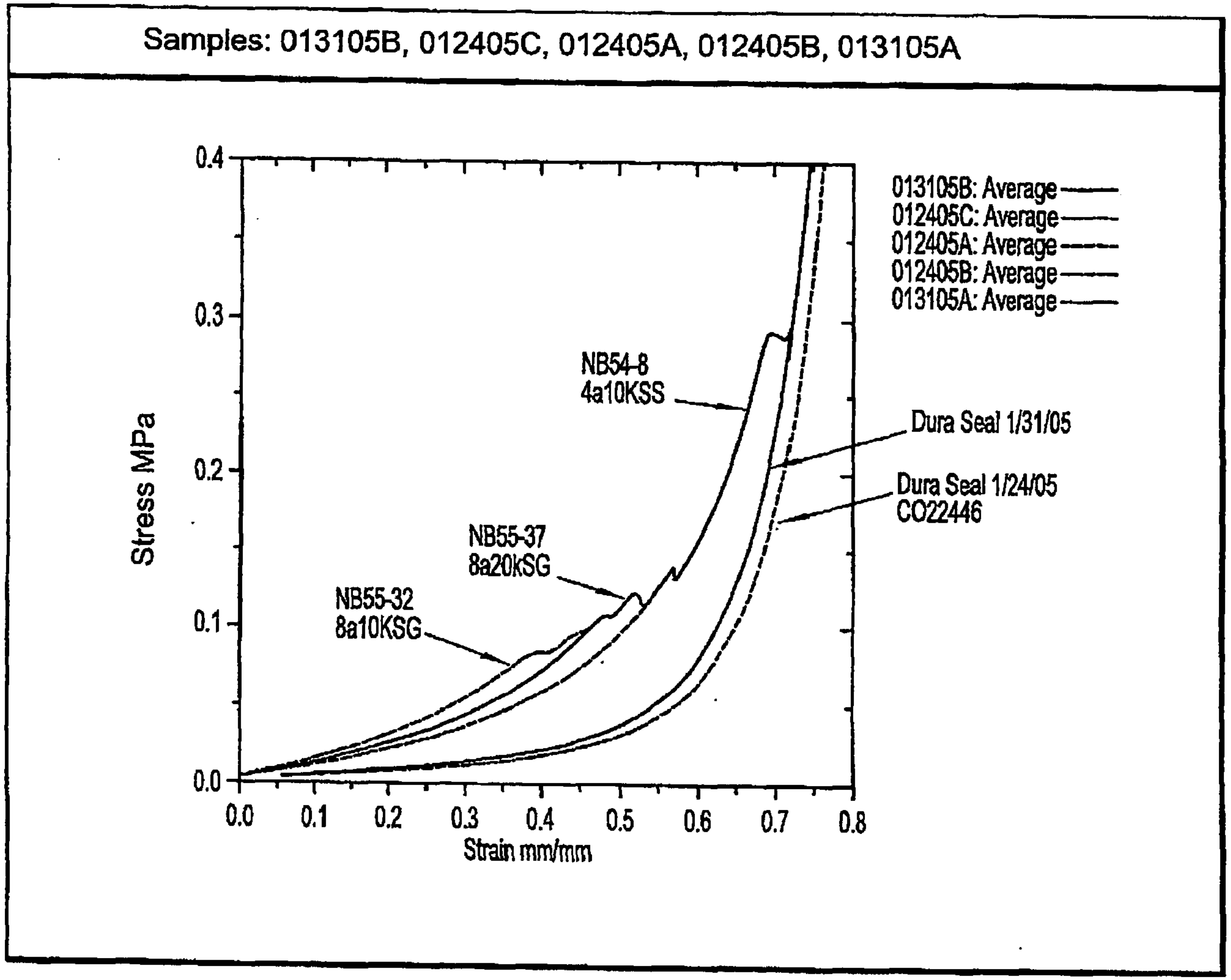


**FIG. 2:** Compressive strength of various hydrogel formulations.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 71402807 A
- US 6656200 B
- US 5874500 B
- US 5543441 B
- US 5514379 B
- US 5410016 B
- US 5162430 B
- US 5324775 B
- US 5752974 B
- US 5550187 B
- US 4938763 A, Dunn
- US 5100992 A
- US 4826945 A, Cohn
- US 4741872 A
- US 5160745 A, De Luca
- US 5410016 A, Hubbell
- US 4874368 A
- US 4631055 A
- US 4735616 A
- US 4359049 A
- US 4978336 A
- US 5116315 A
- US 4902281 A
- US 4932942 A
- US 6179862 A
- US 6673093 A
- US 6152943 A
- US 7009034 B